# EUROPEAN PATENT SPECIFICATION

(11) **EP 3 979 977 B1**
(45) Date of publication and mention of the grant of the patent: **27.09.2023**
(21) Application number: 20714925.3
(22) Date of filing: 17.03.2020
(51) Int. Cl.: A61K 33/30, A61K 8/25, A61K 8/27, A61K 8/46, A61K 9/06, A61P 9/12, A61Q 11/00

(54) **ORAL CARE COMPOSITION COMPRISING A ZINC SALT FOR USE AS A MEDICAMENT FOR LOWERING BLOOD PRESSURE**
ZAHNPFLEGEMITTELZUSAMMENSETZUNG MIT EINEM ZINKSALZ ZUR VERWENDUNG ALS BLUTDRUCKSENKENDES MEDIKAMENT
COMPOSITION DE SOINS BUCCO-DENTAIRES COMPRENANT UN SEL DE ZINC POUR UTILISATION COMME MÉDICAMENT POUR BAISSER LA TENSION ARTÉRIELLE

(30) Priority: 06.06.2019 EP 19178621
(43) Date of publication of application: 13.04.2022
(73) Proprietor: UNILEVER GLOBAL IP LIMITED, Wirral Merseyside CH62 4ZD (GB); Unilever IP Holdings B.V., 3013 AL Rotterdam (NL)
(72) Inventor: ADAMS, Suzanne, Elizabeth, Wirral Merseyside CH63 3JW (GB); ARNOLD, David, Stanley, Wirral Merseyside CH63 3JW (GB); CAWLEY, Andrew, Kenneth, Wirral Merseyside CH63 3JW (GB); GREEN, Alison, Katharine, Wirral Merseyside CH63 3JW (GB); HOPTROFF, Michael, John, Wirral Merseyside CH63 3JW (GB); HUNT, Joanne, Elizabeth, Wirral Merseyside CH63 3JW (GB); MARRIOTT, Robert, Edward, Wirral Merseyside CH63 3JW (GB); SLOMKA, Vera, Wirral Merseyside CH63 3JW (GB)
(74) Representative: Tansley, Sally Elizabeth
(86) International application number: PCT/EP2020/057233
(87) International publication number: WO 2020/244822

(56) References cited:
- EP-A1- 2 915 432
- WO-A1-2016/193067
- CN-A- 102 294 032
- CN-A- 104 522 777
- CN-A- 106 361 768
- CN-A- 107 048 329
- US-A1- 2017 367 953
- US-B1- 7 169 290
- S. A. MUHAMMAD ET AL: "Effect of Antioxidant Mineral Elements Supplementation in the Treatment of Hypertension in Albino Rats", OXIDATIVE MEDICINE AND CELLULAR LONGEVITY, vol. 2012, 2012, pages 1-8, XP055710020, US ISSN: 1942-0900, DOI: 10.1155/2012/134723
- NATHAN S BRYAN ET AL: "Nitrate-Reducing Oral Bacteria: Linking Oral and Systemic Health", 2017, NITRITE AND NITRATE IN HUMAN HEALTH AND DISEASE; 2ND ED, HUMANA PRESS, CHAM, PAGE(S) 21 - 31, XP009516062, ISBN: 978-3-319-46187-8 [retrieved on 2017-02-03] cited in the application the whole document
- MP HEZEL E WEITZBERG: "The oral microbiome and nitric oxide homoeostasis", ORAL DISEASES, STOCKTON PRESS, BASINGSTOKE, GB, vol. 21, no. 1, 2015, pages 7-16, XP009516060, ISSN: 1354-523X, DOI: 10.1111/ODI.12157 [retrieved on 2013-06-28] cited in the application

## Description

The present invention relates to oral care compositions, in particular to oral care compositions comprising zinc.

### Background

Zinc salts have been used in some oral care compounds for a number of years.

US 5 470 561 discloses an anti-plaque mouthwash comprising a zinc salt and triclosan. The composition may also comprise glycine and has a pH of between 4 and 8, preferably between 5 and 7, the preferred pH being 6.

GBA-2 052 978 (Unilever) discloses a toothpaste comprising zinc salts with glycine and having a pH from 4.5 to 8.0.

Toothpaste compositions comprising zinc are disclosed in WO0612977. The toothpaste comprises water, a zinc salt, a chelating agent for the zinc. US2017/367953 discloses oral care compositions comprising zingerone, a stannous ion source, zinc oxide, and zinc citrate.

US 5 632 972 (Williams) discloses a method for minimising damage to gingival and periodontal tissue by delivering a first component comprising zinc and a second component comprising a bicarbonate.

Zinc salts are disclosed in oral care tablets in WO16156157.

US2014170084 discloses that certain zinc salts when added to toothpastes comprising catechins prevent darkening of the colour of the toothpaste.

WO2016/193067 discloses cinnamaldehyde supplemented by zinc, and the combination can be effective to increase at least one of energy expenditure, sympathetic nervous system activity, or fat oxidation

The role of nitrites and nitrates in the human body are discussed in "Chapter 3: The Nitrate-Nitrite-Nitric Oxide Pathway in Mammals" In: "Nitrite and Nitrate in Human Health and Disease", ISBN: 3-319-46187-7 pages 21-48.

In Muhammad, S A et al. "Effect of antioxidant mineral elements supplementation in the treatment of hypertension in albino rats." Oxidative medicine and cellular longevity vol. 2012 (2012): 134723. doi:10.1155/2012/134723, oral supplementation with zinc sulphate was shown to lower the blood pressure of salt-loaded hypertensive rats.

However, the present application has found that the presence of zinc in an oral care composition can deliver a benefit of the body in addition to oral care benefits.

### Detailed Description of the Invention

The present invention relates to an oral care dentifrice composition comprising a zinc salt for use in lowering blood pressure.

The present application has found that a composition can enhance the formation of nitrate to nitrite in the oral cavity, it is highly preferable if it does this by enhancing the activity of nitrate reductase, particularly preferred is Enzyme Commission number: [EC:1.7.5.1].

Further described is a composition comprising a zinc salt to boost the expression of the Kegg Orthologies K00370, K00371, K00373, K00374.

In the above it should be noted that the K numbers relate to the KO (KEGG Orthology). KO is an online tool developed by Kyoto University, Japan and provides maps of metabolic pathways and the genes involved in these pathways. It is used widely by bioinformaticians for biological interpretation of genome sequence data.

Enhanced nitrate reductase activity and hence increased nitrite levels has been cited in the academic literature (Oral diseases (2015) 21 7-16; Nitrite and Nitrate in Human Health and Disease 2nd edition, chapter 3) to have associated health benefits such as promoting cardiovascular health and or vasodilation, in particular reducing blood pressure.

The zinc salt is preferably present at from 0.01 to 5% by weight of the total composition, more preferably 0.1 to 4 wt%, most preferably from 0.5 to 3.0wt%.

Compositions according to the invention preferably comprise water-soluble or sparingly water-soluble sources of zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate. Zinc citrate is particularly preferred.

The composition of the invention is used to clean the surfaces of the oral cavity and is known as an oral care composition.

Accordingly, product forms for compositions of the invention are those which are suitable for brushing and/or rinsing the surfaces of the oral cavity.

The composition of the invention is in the form of a dentifrice. The term "dentifrice" denotes an oral composition which is used to clean the surfaces of the oral cavity. Such a composition is not intentionally swallowed for purposes of systemic administration of therapeutic agents, but is applied to the oral cavity, used to treat the oral cavity and then expectorated. Typically, such a composition is used in conjunction with a cleaning implement such as a toothbrush, usually by applying it to the bristles of the toothbrush and then brushing the accessible surfaces of the oral cavity.

Preferably the dentifrice/toothpaste is in the form of an extrudable semi-solid such as a cream, paste or gel (or mixture thereof).

A composition according to the invention (such as a dentifrice/toothpaste) will generally contain further ingredients to enhance performance and/or consumer acceptability, in addition to the ingredients specified above.

Compositions according to the invention, particularly toothpastes, preferably comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition.

Preferably the composition, particularly a toothpaste, comprises a silica based abrasive. The preferred abrasive silicas used in the present invention is a silica with a low refractive index. It may be used as the sole abrasive silica, or in conjunction with a low level of other abrasive silicas, e.g. those according to EP 236070. Typical examples of suitable low refractive index abrasive silicas (e.g. having an R.I. of between 1.435 and 1.445) are Tixosil 63 and 73 ex Rhone Poulenc; Sident 10 ex Degussa; Zeodent 113 ex Zeofinn; Zeodent 124 ex Huber, Sorbosil AC 77 ex Crosfield Chemicals (having an R.I. of approximately 1.440). The amount of these silicas in the total composition generally ranges from 5-60% by weight, usually 5-20% by weight.

The composition, particularly if a toothpaste, preferably comprises an inorganic or a natural or synthetic thickener or gelling agent in proportions of about 0.10 to about 15% by weight depending on the material chosen. These proportions of thickeners in the dentifrice compositions of the present invention form an extrudable, shape-retaining product which can be squeezed from a tube onto a toothbrush and will not fall between the bristles of the brush but rather, will substantially maintain its shape thereon. Suitable thickeners or gelling agents useful in the practice of the present invention include inorganic thickening silicas such as amorphous silicas available from Huber Corporation under the trade designation Zeodent 165, Irish moss, iota-carrageenan, gum tragacanth, and polyvinylpyrrolidone.

Compositions according to the invention preferably comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:
Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment in the composition is from 0.01 wt% to 3 wt, more preferably from 0.02 to 2 wt%.

If the composition is a toothpaste it may be a dual phase paste, with the whitening pigments present in one phase.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof.

Compositions of the invention may comprise fluoride sources such as sodium fluoride, stannous fluoride, sodium monofluorophosphate, zinc ammonium fluoride, tin ammonium fluoride, calcium fluoride, cobalt ammonium fluoride and mixtures thereof.

In one embodiment a preferred class of oral care active for inclusion in the compositions of the invention includes agents for the remineralisation of teeth. The term "remineralisation" in the context of the present invention means the *in situ* generation of hydroxyapatite on teeth.

A specific example of a suitable agent for the remineralisation of teeth is a mixture of a calcium source and a phosphate source which, when delivered to the teeth results in the *in situ* generation of hydroxyapatite on teeth.

Illustrative examples of the types of calcium source that may be used in this context (hereinafter termed "remineralising calcium sources") include, for example, calcium phosphate, calcium gluconate, calcium oxide, calcium lactate, calcium glycerophosphate, calcium carbonate, calcium hydroxide, calcium sulphate, calcium carboxymethyl cellulose, calcium alginate, calcium salts of citric acid, calcium silicate and mixtures thereof. Preferably the remineralising calcium source is calcium silicate.

The amount of remineralising calcium source(s) (e.g. calcium silicate) in the composition of the invention typically ranges from 1 to 30%, preferably from 5 to 20% by total weight remineralising calcium source based on the total weight of the oral care composition.

Illustrative examples of the types of phosphate source that may be used in this context (hereinafter termed "remineralising phosphate sources") include, for example, monosodium dihydrogen phosphate, disodium hydrogen phosphate, sodium pyrophosphate, tetrasodium pyrophosphate, sodium tripolyphosphate, sodium hexametaphosphate, potassium dihydrogenphosphate, trisodium phosphate, tripotassium phosphate and mixtures thereof. Preferably the remineralising phosphate source is a mixture of trisodium phosphate and sodium dihydrogen phosphate.

The amount of remineralising phosphate source(s) (e.g. trisodium phosphate and sodium dihydrogen phosphate) in the composition of this invention typically ranges from 2 to 15%, preferably from 4 to 10% by total weight remineralising phosphate source based on the total weight of the oral care composition.

Mixtures of any of the above described materials may also be used.

The composition according the invention will comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
amino acids such as arginine;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
Humectants such as glycerol, sorbitol, propyleneglycol, xylitol, lactitol etc.;
binders and thickeners such as sodium carboxymethyl-cellulose, hydroxyethyl cellulose (Natrosol^{®}), xanthan gum, gum arabic etc. as well as synthetic polymers such as polyacrylates and carboxyvinyl polymers such as Carbopol^{®};
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

### Examples will now be illustrated by the following non-limiting Examples:

### Examples

A clinical study was conducted to determine the effect of use of a product containing zinc citrate trihydrate compared to a control product on the oral microbiome. Following a 4-week washout period subjects used one of the Examples detailed below for a 6-week period with the dental plaque collected at baseline and 6 weeks. The samples were collected into an RNA protect solution to stabilise the RNA prior to analysis of microbiome gene activity (transcriptomics).

**Table 1 - Toothpaste formulations used in the washout and test phases of the study**

| **Ingredient** | **Example A wt%** | **Example 1 wt%** | **Wash out product** |
|---|---|---|---|
| Sorbitol | 45 | 45 | 45 |
| Hydrated silica | 18 | 18 | 17.5 |
| PEG32 | 2 | 2 | 5 |
| Sodium Lauryl sulphate | 1.8 | 1.8 | 1.5 |
| Titanium dioxide | 1 | 1 | 1 |
| Carboxy methyl cellulose | 0.8 | 0.8 | 0.63 |
| Sodium Fluoride | 0.32 | 0.32 | 0.32 |
| Zinc Citrate Trihydrate | 0 | 2 | 0 |
| Water and minors | To 100 | To 100 | To 100 |

Plaque samples were pooled for each product and time point and split into three to provide replicates for RNA extraction and analysis, in total yielding 18 samples. The RNA was extracted using the Qiagen AllPrep kit. The RNA samples were subjected to RNA sequencing. Following depletion of the ribosomal RNA (rRNA) to allow sequencing of only the messenger RNA (mRNA) to focus on the more informative parts of the transcriptome, libraries were sequenced on an Illumina HiSeq 4000 platform.

RNA sequence data was processed using a bioinformatics pipeline (Eagle Genomics) to determine microbiome community activity via analysis of gene expression (mRNA) using the Human2 database. Analysis of individual genes was conducted using a commercial software package JMP-

**Table 2 - Relative abundance (%) of genes associated with nitrate reductase (E.C: 1.7.5.1) for the Control and Zinc pastes at different timepoints (mean (std dev)).**

| | **Baseline** | **Week 6** | **Baseline week 6** |
|---|---|---|---|
| Example A | 0.0210(0.0034) | 0.0376(0.0013) | P=0.02457 |
| Example 1 | 0.0183(0.0036) | 0.0646 (0.0013) | P=0.00106 |
| | P=0.39256 | P=0.00001 | |

Analysis of nitrate reductase (E.C: 1.7.5.1) gene expression associated with the Examples was conducted as described above showed the following:. A significant increase (T-test, p value < 0.05) in the expression of this gene was observed for the zinc product compared to the control product at 6 weeks of product use.

The Examples demonstrate use of a zinc containing toothpaste significantly increases the expression of the gene involved in the conversion of nitrate to nitrite compared to a control toothpaste.

## Claims

1. An oral care dentifrice composition comprising a zinc salt for use as a medicament for lowering blood pressure.

2. An oral care dentifrice composition for use according to claim 1 to enhance the formation of nitrate to nitrite in the oral cavity.

3. An oral care dentifrice composition for use according to any preceding claim in which the zinc salt is a zinc citrate.

4. An oral care dentifrice composition for use according to any preceding claim in which the level of zinc salt is from 0.1 to 4 wt% of the total composition.

5. An oral care dentifrice composition for use according to any preceding claim that further comprises silica.

6. An oral care dentifrice composition for use according to any preceding claim that further comprises an anionic and/or nonionic cleansing surfactant.

## Patentansprüche

1. Zahnpflegemittelzusammensetzung, umfassend ein Zinksalz zur Verwendung als Arzneimittel zur Senkung des Blutdrucks.

2. Zahnpflegemittelzusammensetzung zur Verwendung nach Anspruch 1 zur Verbesserung der Bildung von Nitrat zu Nitrit in der Mundhöhle.

3. Zahnpflegemittelzusammensetzung zur Verwendung nach einem vorhergehenden Anspruch, in welcher das Zinksalz ein Zinkcitrat ist.

4. Zahnpflegemittelzusammensetzung zur Verwendung nach einem vorhergehenden Anspruch, in der der Gehalt des Zinksalzes 0,1 bis 4 Gew.-% der Gesamtzusammensetzung beträgt.

5. Zahnpflegemittelzusammensetzung zur Verwendung nach einem vorhergehenden Anspruch, die ferner Siliciumdioxid enthält.

6. Zahnpflegemittelzusammensetzung zur Verwendung nach einem vorhergehenden Anspruch, die ferner ein anionisches und/oder nichtionisches Reinigungstensid enthält.

## Revendications

1. Composition de dentifrice pour le soin oral comprenant un sel de zinc pour une utilisation comme un médicament pour abaisser la pression sanguine.

2. Composition de dentifrice pour le soin oral pour une utilisation selon la revendication 1 pour promouvoir la formation de nitrate en nitrite dans la cavité orale.

3. Composition de dentifrice pour le soin oral pour une utilisation selon l'une quelconque des revendications précédentes dans laquelle le sel de zinc est un citrate de zinc.

4. Composition de dentifrice pour le soin oral pour une utilisation selon l'une quelconque des revendications précédentes dans laquelle la teneur en sel de zinc est de 0,1 à 4 % en masse de la composition totale.

5. Composition de dentifrice pour le soin oral pour une utilisation selon l'une quelconque des revendications précédentes qui comprend de plus de la silice.

6. Composition de dentifrice pour le soin oral pour une utilisation selon l'une quelconque des revendications précédentes qui comprend de plus un tensioactif de nettoyage anionique et/ou non ionique.
